# EUROPEAN PATENT APPLICATION

(11) **EP 4 516 913 A1**
(43) Date of publication of application: **05.03.2025**
(21) Application number: 23796711.2
(22) Date of filing: 21.04.2023
(51) Int. Cl.: C12N 15/86, C07K 14/475, A61K 48/00, A61K 38/18, A61K 9/00, A61P 25/28, A61P 25/02

(54) **AAV VECTOR OPTIMIZED FOR INTRATHECAL ADMINISTRATION INTO WHICH HEPATOCYTE GROWTH FACTOR GENE IS INTRODUCED**

(30) Priority: 27.04.2022 KR 20220052369
(71) Applicant: Helixmith Co., Ltd, Seoul 07794 (KR)
(72) Inventor: LEE, Junghun, Seoul 06280 (KR); NHO, Boram, Seoul 06520 (KR); YU, Seungshin, Seoul 04732 (KR)
(74) Representative: J A Kemp LLP
(86) International application number: PCT/KR2023/005447
(87) International publication number: WO 2023/211064

(57) **Abstract**

The present invention relates to an adeno-associated virus (AAV) vector optimized for intrathecal administration into which a hepatocyte growth factor gene is introduced. The AAV vector of the present invention and AAV particles produced through the vector have excellent productivity of AAV particles comprising a hepatocyte growth factor gene, and when administered intrathecally to a mammal, the expression efficiency of hepatocyte growth factor is also very high. Therefore, the AAV vector of the present invention and the AAV particles induce the expression of hepatocyte growth factor protein when administered intrathecally, and thus can be effectively used in the prevention or treatment of related diseases.

## Description

### Technical Field

This patent application claims the benefit of and priority to Korean Patent Application No. 10-2022-0052369, filed on April 27, 2022, with the Korean Intellectual Property Office, the disclosures of which are incorporated herein by reference.

The present disclosure relates to an adeno-associated virus (AAV) vector, optimized for intrathecal administration, with a hepatocyte growth factor (HGF) gene introduced thereinto.

### Background Art

Gene therapy is a method that utilizes recombinant gene technology to deliver therapeutic genes into a patient's cells, thereby inducing genetic modification of target cells or expressing specific proteins to treat genetic disorders and intractable diseases. The substance used to deliver the gene into the body is called a carrier or vector, and vectors are broadly classified into viral vectors and non-viral vectors. Representative viral vectors include retroviruses and adenoviruses, while non-viral vectors include naked DNA and liposomes.

The present inventors have developed an adeno-associated virus (AAV) for use in gene therapy, with a hepatocyte growth factor (HGF) gene introduced thereinto (Lee et al., BBRC 517:452-457, 2019; Lee et al., Acta Neuropathologica Communications 7:14, 2019). However, conventional AAVs have limitations such as low productivity and significantly low levels of protein expression when administered intrathecally. Therefore, there has been a need to develop new AAV vectors optimized for intrathecal administration, improving both productivity and protein expression levels.

Throughout this specification, numerous papers and patent documents are referenced and citations are indicated. The disclosures of the cited papers and patent documents are incorporated by reference in their entirety into this specification to more clearly describe the level of skill in the art and the content of the present disclosure.

### Disclosure of Invention

### Technical Problem

Leading to the present disclosure, thorough and intensive research conducted by the present inventors to develop a new adeno-associated virus vector (AAV) with improved productivity and protein expression levels resulted in the finding that the productivity of AAV particles is significantly enhanced when combining specific promoters, AAV serotypes, and polyadenylation sequences and the protein expression levels are greatly improved when these AAV particles are administered intrathecally.

Accordingly, the present disclosure aims to provide an AAV vector, AAV particles, and a pharmaceutical composition containing same, wherein the AAV vector is optimized for intrathecal administration, with a hepatocyte growth factor (HGF) gene introduced thereinto.

Other objectives and advantages of the present disclosure will become more apparent from the detailed description, claims, and drawings provided below.

### Solution to Problem

Provided according to one aspect of the present disclosure is an adeno-associated virus (AAV) vector carrying nucleic acid molecules comprising:
(a) a Cytomegalovirus (CMV) promoter sequence or an Elongation Factor 1-alpha (EF-1alpha) promoter sequence; and
(b) a polynucleotide sequence encoding hepatocyte growth factor (HGF), operatively linked to the promoter sequence.

The present inventors conducted extensive research to develop a new adeno-associated virus (AAV) vector with enhanced productivity and protein expression levels. As a result, the inventors confirmed that the productivity of AAV particles is significantly increased when combining specific promoters, AAV serotypes, and polyadenylation sequences. Furthermore, the inventors identified that the protein expression levels are greatly improved when these AAV particles are administered intrathecally.

As used herein, the term "vector" refers to a non-chromosomal nucleic acid comprising an intact replicon such that the vector may be replicated when placed within a cell, for example by a process of transformation. Vectors may be viral or non-viral. Examples of viral vectors include retroviruses, adenoviruses, adeno-associated virus (AAV), herpesvirus, bacculoviruses, modified bacculoviruses, papovirus, and the like, but are not limited thereto. Exemplary non-viral vectors for delivering nucleic acid include naked DNA; DNA complexed with cationic lipids, alone or in combination with cationic polymers; anionic and cationic liposomes; DNA-protein complexes and particles comprising DNA condensed with cationic polymers such as heterogeneous polylysine, defined-length oligopeptides, and polyethylene imine, in some cases contained in liposomes; and the use of ternary complexes comprising a virus and polylysine-DNA.

A "viral vector" is defined as a recombinantly produced virus or viral particle that comprises a polynucleotide to be delivered into a host cell, either in vivo, ex vivo or in vitro. Examples of viral vectors include retroviral vectors, AAV vectors, lentiviral vectors, adenovirus vectors, alphavirus vectors and the like. Alphavirus vectors, such as Semliki Forest virus-based vectors and Sindbis virus-based vectors, have also been developed for use in gene therapy and immunotherapy (see, Schlesinger and Dubensky (1999) Curr. Opin. Biotechnol. 5:434-439 and Ying, et al. (1999) Nat. Med. 5(7):823-827).

The vector may include nucleic acid sequences that allow replication in host cells, such as an origin of replication. In some cases, the vector may lack nucleic acid sequences allowing replication in host cells. Additionally, the vector may include target genes, one or more selectable marker genes, other known genetic elements in the art, or any other suitable inserts.

In an embodiment of the present disclosure, the term "adeno-associated virus" or "AAV" refers to a member belonging to the genus dependoparvovirus, family Parvoviridae. Multiple serotypes of this virus are known to be suitable for gene delivery. At least 11 sequentially numbered AAV serotypes have been identified in the field: AAV1, AAV2, AAV4, AAV5, AAV6, AAV8, AAV9, AAV10, AAV11, AAV12, and AAV13.

In an embodiment of the present disclosure, the AAV serotype includes the following variant serotypes: AAV-DJ, AAV-DJ/8, AAV-PHP.Eb, AAV-PHP.S, and AAV rh10.

As used herein, the term "AAV vector" refers to a vector that includes at least one polynucleotide of interest (or transgene) flanked by inverted terminal repeat sequences (ITR sequences) on both sides. AAV vectors can be replicated and packaged into infectious viral particles when transfected into host cells that express viral DNA replication (rep) and capsid formation (cap) gene products.

The term "AAV virion," "AAV viral particle," or "AAV vector particle" refers to a viral particle composed of at least one AAV capsid protein and an encapsidated polynucleotide AAV vector.

The "AAV" is a replication-deficient parvovirus, the single-stranded DNA genome of which is approximately 4.7 kb in length including two nucleotide inverted terminal repeat sequences (ITR sequences). There are multiple AAV serotypes. The nucleotide sequences of the genomes of the AAV serotypes are known. For example, the complete genome of AAV-1 is available under GenBank accession number NC_002077; the complete genome of AAV-2 is provided in GenBank accession number NC_001401 and Srivastava et al., J. Virol., 45: 555-564 (1983); the complete genome of AAV-3 is provided in GenBank accession number NC_1829; the complete genome of AAV-4 is provided in GenBank accession number NC_001829; the genome of AAV-5 is provided in GenBank accession number AF085716; the complete genome of AAV-6 is provided in GenBank accession number NC_001862; at least portions of the genomes of AAV-7 and AAV-8 are provided GenBank accession numbers AX753246 and AX753249, respectively; the genome of AAV-9 is provided in Gao et al., J. Virol., 78: 6381-6388 (2004); the genome of AAV-10 is provided in Mol. Ther., 13(1): 67-76 (2006); and the genome of AAV-11 is provided in Virology, 330(2): 375-383 (2004).

In an embodiment of the present disclosure, the genome of AAV may be a self-complementary genome. In this regard, cis-acting sequences directing viral DNA replication (rep), encapsidation (cap)/packaging and host cell chromosome integration are contained within the AAV ITR sequence. The rep protein ultimately possesses multiple enzymatic activities that contribute to the replication of the viral genome. The cap gene encodes the three capsid proteins VP1, VP2, and VP3 the expression of which is driven by the p40 promoter. To generate an AAV vector, the rep and cap proteins may be provided in trans.

AAV can be packaged using viral packaging systems, such as retrovirus, adenovirus, herpesvirus, or baculovirus packaging systems.

In an embodiment of the present disclosure, packaging is achieved using a helper virus or helper plasmid along with a cell line. The helper virus or helper plasmid contains elements and sequences (e.g., E4, E2a, and VA) that facilitate the delivery of genetic material into cells.

A helper plasmid may include, for example, at least one viral helper DNA sequence derived from a replication-incompetent viral genome encoding in trans all virion proteins required to package a replication-incompetent AAV, and for producing virion proteins capable of packaging the replication-incompetent AAV at high titer, without the production of replication-competent AAV.

In the packaging process, the helper plasmids and the plasmids encoding the AAV viral proteins are transiently co-transfected into mammalian cells that are capable of producing virus, such as human embryonic kidney cells, for example, 293 cells (ATCC No. CRL1573, ATCC, Rockville, Md.) to produce high titer recombinant retrovirus-containing supernatants.

The term "promoter", as used herein, refers to any sequence that regulates the expression of a coding sequence, such as a gene. Promoters may be constitutive, inducible, repressible, or tissue-specific, for example. A "promoter" is a control sequence that is a region of a polynucleotide sequence at which initiation and rate of transcription are controlled.

In an embodiment of the present disclosure, the promoter may be a Rous Sarcoma Virus (RSV) LTR promoter (optionally including an RSV enhancer), a Cytomegalovirus (CMV) promoter, an SV40 promoter, a dihydrofolate reductase promoter, a β-actin promoter, a phosphoglycerate kinase (PGK) promoter, a U6 promoter, or an Elongation Factor 1-alpha (EF-1alpha) promoter, but with no limitations thereto.

In a specific embodiment of the present disclosure, the promoter is a CMV promoter or an EF-1 alpha promoter.

As used herein, the term "nucleic acid molecule" is intended to comprehensively encompass DNA (gDNA and cDNA) and RNA molecules, and the nucleotides, which are the basic building blocks of the nucleic acid molecule, may include not only natural nucleotides but also analogs modified in the sugar or base portions (Scheit, Nucleotide Analogs, John Wiley, New York(1980); Uhlman and Peyman, Chemical Reviews, 90:543-584(1990).

The nucleotide sequence encoding the hepatocyte growth factor (HGF) in the present disclosure is sufficient if it encodes the amino acid sequence of hepatocyte growth factor or its isoforms, and it is obvious to those skilled in the art that the nucleotide sequence is not limited to any specific nucleotide sequence.

This is because even if mutations occur in the nucleotide sequence, the mutated nucleotide sequence may not lead to changes in the protein sequence when expressed, which is referred to as codon degeneracy. Therefore, the nucleotide sequence may include codons that are functionally equivalent or codons that encode the same amino acid (e.g., due to codon degeneracy, six different codons can encode arginine or serine), or codons that encode biologically equivalent amino acids.

Considering the aforementioned biologically equivalent activity of mutations, the nucleic acid molecule of the present disclosure, encoding the amino acid sequence of hepatocyte growth factor, may also be interpreted to include sequences that show substantial identity with such sequences. The substantial identity refers to a homology given to a sequence that, when aligned with any other sequence as closely as possible using an algorithm commonly used in the art, shows at least 60% homology (e.g., 61% , 62%, 63%, 64%, 65%, 66%, 67%, 68%, or 69%), more specifically, at least 70% homology (e.g., 71%, 72%, 73%, 74%, 75%, 76%, 77%, 78%, or 79%), even more specifically, at least 80% homology (e.g., 81%, 82%, 83%, 84%, 85%, 86%, 87%, 88%, or 89%), still more specifically, at least 90% homology (e.g., 91%, 92%, 93%, 94%, 95%, 96%, 97%, 98%, or 99%), and most specifically, at least 95% homology (e.g., 95%, 96%, 97%, 98%, or 99%). Any integer between 60% and 100%, as well as decimal values between these percentages, is included in the scope of the present disclosure in terms of % homology.

Alignment methods for sequence comparison are well known in the art. Various methods and algorithms for alignment are described in Smith and Waterman, Adv. Appl. Math. 2:482

(1981); Needleman and Wunsch, J. Mol. Bio. 48:443 (1970); Pearson and Lipman, Methods in Mol. Biol. 24: 307-31 (1988); Higgins and Sharp, Gene 73:237-44 (1988); Higgins and Sharp, CABIOS 5:151-3 (1989); Corpet et al., Nuc. Acids Res. 16:10881-90 (1988); Huang et al., Comp. Appl. BioSci. 8:155-65 (1992); and Pearson et al., Meth. Mol. Biol. 24:307-31 (1994). The NCBI Basic Local Alignment Search Tool (BLAST) (Altschul et al., J. Mol. Biol. 215:403-10 (1990)) is accessible via National Center for Biological Information (NCBI) and can be used in conjunction with sequence analysis programs such as blastp, blastn, blastx, tblastn, and tblastx over the internet. BLAST can be accessed via the BLAST page on the NCBI website. The method for sequence homology comparison using this program can be found on the BLAST help page of the NCBI website.

In an embodiment of the present disclosure, the AAV vector of the present disclosure may include a polynucleotide sequence encoding any polypeptide with a different function instead of the aforementioned polynucleotide sequence encoding hepatocyte growth factor.

As used herein, the term "operatively linked" refers to a functional connection between a nucleic acid expression control sequence (e.g., a promoter, signal sequence, or an array of transcription factor binding sites) and another nucleic acid sequence, such that the control sequence regulates the transcription and/or translation of the other nucleic acid sequence.

In an embodiment of the present disclosure, the hepatocyte growth factor is a hepatocyte growth factor (HGF) isoform. The term "HGF isoform", as used herein, refers to an HGF polypeptide having at least 80%, such as 80%, 85%, 90%, or 95% identity with the amino acid sequence of a naturally occurring HGF found in animals, including any allelic variant. For example, the term "HGF isoform" is intended to comprehensively encompass both the normal or wild-type HGF and various variants of HGF, such as splicing variants and deletion variants.

In an embodiment of the present disclosure, the hepatocyte growth factor isoform may be a full-length hepatocyte growth factor (flHGF), a deleted variant hepatocyte growth factor (dHGF), or a combination thereof, but with no limitations thereto.

The term "flHGF", as used herein, refers to the amino acid sequence from amino acids 1 to 728 of an animal HGF sequence, including, in an embodiment, the amino acid sequence from amino acids 1 to 728 of a mammalian HGF sequence, and in another embodiment, the amino acid sequence from amino acids 1 to 728 of a human HGF sequence.

As used herein, the term "dHGF" refers to a deleted variant of the HGF protein generated by alternative splicing of the HGF gene in animals, including mammals. In another embodiment of the present disclosure, dHGF refers to a human HGF composed of 723 amino acids, in which five amino acids (F, L, P, S, and S) are deleted from the first kringle domain of the alpha chain of the full-length HGF sequence.

In an embodiment of the present disclosure, the HGF isoform may be encoded by a hybrid HGF gene that simultaneously expresses two or more different types of isoforms, such as flHGF and dHGF.

In an embodiment of the present disclosure, the hybrid HGF gene includes the sequences corresponding to exons 1 to 4 of the human HGF gene; intron 4 or a fragment thereof from the human HGF gene; and the sequences corresponding to exons 5 to 18 of the human HGF gene.

In a specific embodiment of the present disclosure, the full-length hepatocyte growth factor includes the amino acid sequence of SEQ ID NO: 1, and the deleted variant hepatocyte growth factor includes the amino acid sequence of SEQ ID NO: 2, but with no limitations thereto.

In an embodiment of the present disclosure, the hybrid HGF gene that includes intron 4 is 7,113 base pairs in length and includes the nucleotide sequence of SEQ ID NO: 3.

In another embodiment of the present disclosure, the hybrid HGF gene may optionally include a fragment of intron 4 between exons 4 and 5 of HGF cDNA.

In an embodiment of the present disclosure, the polynucleotide sequence of the gene inserted into the AAV vector may be modified to achieve stable expression of the polypeptide encoded thereby. Such modifications may include shortening the length of the inserted polynucleotide sequence by including only a portion of the intron sequence instead of the entire intron sequence.

In a more specific embodiment of the present disclosure, the hybrid HGF gene includes a nucleotide sequence selected from the group consisting of the nucleotide sequences of SEQ ID NOS: 5 to 9 and SEQ ID NO: 19. The nucleotide sequences of SEQ ID NOS: 5 to 8 represent HGF-X7-d1 to HGF-X7-d4 genes, which are truncated versions of the nucleotide sequence of HGF-X7 represented by SEQ ID NO: 4 and contain only part of the intron 4 sequence of the HGF gene.

The "HGF isoform" and "hybrid HGF gene" (e.g., HGF-X7) of the present disclosure are reported in WO 2003/078568, the content of which is incorporated herein by reference in its entirety.

In an embodiment of the present disclosure, the polynucleotide sequence encoding the hepatocyte growth factor or an isoform thereof is codon-optimized.

In a specific embodiment of the present disclosure, the codon-optimized polynucleotide sequence encoding hepatocyte growth factor or an isoform thereof includes the nucleotide sequence of SEQ ID NO: 9.

In an embodiment of the present disclosure, the polynucleotide sequence encoding hepatocyte growth factor or an isoform thereof may have at least 90%, 91%, 92%, 93%, 94%, 95%, 96%, 97%, 98%, or 99% identity to a nucleotide sequence selected from the group consisting of the nucleotide sequences of SEQ ID NOS: 4 to 9 or SEQ ID NO: 19, but with no limitations thereto.

In an embodiment of the present disclosure, the AAV vector has a serotype selected from the group consisting of AAV1, AAV2, AAV4, AAV5, AAV6, AAV8, AAV9, AAV10, AAV11, AAV12, AAV13, AAV-DJ, AAV-DJ/8, AAV-PHP.Eb, AAV-PHP.S, and AAV rh10, but is not limited thereto.

In an embodiment of the present disclosure, the vector is an AAV vector with a serotype of AAV1 or AAV9.

In an embodiment of the present disclosure, the CMV promoter sequence may include the nucleotide sequence of SEQ ID NO: 10 or 11, or a nucleotide sequence with an identity of at least 90%, 91%, 92%, 93%, 94%, 95%, 96%, 97%, 98%, or 99% identity with the nucleotide sequence of SEQ ID NO: 10 or 11.

In an embodiment of the present disclosure, the CMV promoter sequence may include a fragment of the entire CMV promoter sequence. The fragment may contain the minimal functional sequence of the CMV promoter. By way of example, the CMV promoter sequence may include the nucleotide sequence of SEQ ID NO: 10 and may further include sequences from the 5'-end or 3'-end of the CMV promoter sequence relative to the fragment.

In an embodiment of the present disclosure, the EF-1 alpha promoter sequence may include the nucleotide sequence of SEQ ID NO: 12, or a nucleotide sequence with an identity of at least 90%, 91%, 92%, 93%, 94%, 95%, 96%, 97%, 98%, or 99% with the nucleotide sequence of SEQ ID NO: 12. Any integer between 90% and 100%, as well as decimal values between these percentages, is included in the scope of the present disclosure in terms of % identity.

In an embodiment of the present disclosure, the nucleic acid molecule further includes a posttranscriptional regulatory element (PRE).

In a specific embodiment of the present disclosure, the posttranscriptional regulatory element includes the Woodchuck hepatitis virus posttranscriptional regulatory element (WPRE), but with no limitations thereto.

In an embodiment of the present disclosure, the nucleic acid molecule further includes a polyadenylation sequence.

In an embodiment of the present disclosure, the polyadenylation sequence includes a human growth hormone (hGH) polyadenylation sequence, a bovine growth hormone (bGH) polyadenylation sequence, an SV40 polyadenylation sequence, or a combination thereof, but is not limited thereto.

In a specific embodiment of the present disclosure, the human growth hormone (hGH) polyadenylation sequence includes the nucleotide sequence of SEQ ID NO: 13 or SEQ ID NO: 14, or a nucleotide sequence with an identity of at least 90%, 91%, 92%, 93%, 94%, 95%, 96%, 97%, 98%, or 99% with the nucleotide sequence of SEQ ID NO: 13 or SEQ ID NO: 14. Any integer between 90% and 100%, as well as decimal values between these percentages, is included in the scope of the present disclosure in terms of % identity.

In an embodiment of the present disclosure, the human growth hormone (hGH) polyadenylation sequence may include a fragment of the entire hGH polyadenylation sequence. The fragment may contain the minimal functional sequence of the hGH polyadenylation sequence. For example, the hGH polyadenylation sequence may include the nucleotide sequence of SEQ ID NO: 13, and may further include sequences from the 5'-end or 3'-end of the hGH polyadenylation sequence relative to the fragment.

In a specific embodiment of the present disclosure, the bovine growth hormone (bGH) polyadenylation sequence includes the nucleotide sequence of SEQ ID NO: 15 or 16, or a nucleotide sequence with an identity of at least 90%, 91%, 92%, 93%, 94%, 95%, 96%, 97%, 98%, or 99% with the nucleotide sequence of SEQ ID NO: 15 or 16. Any integer between 90% and 100%, as well as decimal values between these percentages, is included in the scope of the present disclosure in terms of % identity.

In an embodiment of the present disclosure, the bovine growth hormone (bGH) polyadenylation sequence may include a fragment of the entire bGH polyadenylation sequence. The fragment may contain the minimal functional sequence of the bGH polyadenylation sequence. For example, the bGH polyadenylation sequence may include the nucleotide sequence of SEQ ID NO: 15, and may further include sequences from the 5'-end or 3'-end of the bGH polyadenylation sequence relative to the fragment.

In a specific embodiment of the present disclosure, the SV40 polyadenylation sequence includes the nucleotide sequence of SEQ ID NO: 17 or 18, or a nucleotide sequence with an identity of at least 90%, 91%, 92%, 93%, 94%, 95%, 96%, 97%, 98%, or 99% identity with the nucleotide sequence of SEQ ID NO: 17 or 18. Any integer between 90% and 100%, as well as decimal values between these percentages, is included in the scope of the present disclosure in terms of % identity.

In an embodiment of the present disclosure, the SV40 polyadenylation sequence may include a fragment of the entire SV40 polyadenylation sequence. The fragment may contain the minimal functional sequence of the SV40 polyadenylation sequence. For example, the SV40 polyadenylation sequence may include the nucleotide sequence of SEQ ID NO: 17, and may further include sequences from the 5'-end or 3'-end of the SV40 polyadenylation sequence relative to the fragment.

In an embodiment of the present disclosure, the nucleic acid molecule includes two AAV inverted terminal repeat sequences (ITR sequences) flanking the nucleic acid sequence on both sides.

Another aspect of the present disclosure provides an isolated host cell comprising the AAV vector.

So long as it is known in the art and allows the AAV vector of the present disclosure to be continuously cloned and expressed with stability, any host cell is available. Examples of eukaryotic host cells suitable for the vector include yeast cells (Saccharomyce cerevisiae), insect cells, monkey kidney cells (COS7), NSO cells, SP2/0, Chinese hamster ovary (CHO) cells, W138, baby hamster kidney cells (BHK), MDCK, myeloma cells, HuT 78 cells, and HEK-293 cells, but are not limited thereto.

As used herein, the term "transformed", "transduced", or "transfected" refers to pertaining to the delivery or introduction of a foreign nucleic acid into a host cell. The "transformed", "transduced", or "transfected" cells are cells into which a foreign nucleic acid is transformed, transduced, or transfected. Within the scope of the transformed, transduced, or transfected cells, the cells themselves and progeny cells thereof obtained through passages fall.

For eukaryotic host cells, the method of delivering the vector of the present disclosure into host cells may include microinjection (Capecchi, M.R., Cell, 22:479(1980)), calcium phosphate precipitation (Graham, F.L. et al., Virology, 52:456(1973)), electroporation (Neumann, E. et al., EMBO J., 1:841(1982)), liposome-mediated transfection (Wong, T.K. et al., Gene, 10:87(1980)), DEAE-dextran treatment (Gopal, Mol. Cell Biol., 5:1188-1190(1985)), and gene bombardment (Yang et al., Proc. Natl. Acad. Sci., 87:9568-9572(1990)) to inject the vector into the host cells.

The vector introduced into the host cells in the present disclosure can express the proteins required for the production of AAV viral particles within the host cell, and through the action of the expressed proteins and their assembly, AAV viral particles are obtained.

The cultivation is typically performed under aerobic conditions, such as shaking incubation or rotation on a rotary shaker. The cultivation is carried out generally for 5 hours to 7 days at a temperature ranging preferably from 10 to 40°C. The medium is maintained preferably at a pH of 3.0 to 9.0 during cultivation. The pH of the medium can be adjusted using inorganic or organic acids, alkaline solutions, urea, calcium carbonate, ammonia, and the like. If necessary, antibiotics such as ampicillin, streptomycin, chloramphenicol, kanamycin, and tetracycline may be added during cultivation to maintain and express the recombinant vector. If the host cell is transformed with a recombinant expression vector having an inducible promoter, an appropriate inducer may be added to the medium as necessary. For example, if the expression vector contains a lac promoter, isopropyl-beta-D-thiogalactopyranoside (IPTG) can be added, and if it contains a trp promoter, indoleacrylic acid can be added to the medium.

Provided according to another aspect of the present disclosure is an AAV particle comprising the aforementioned AAV vector.

In one embodiment of the present disclosure, the AAV particle is for intrathecal administration.

In an embodiment of the present disclosure, the AAV particle exhibits superior efficiency in expressing the transfected gene of interest (GOI) as a protein upon intrathecal administration.

Provided according to another aspect of the present disclosure is a pharmaceutical composition comprising the AAV particle for the prevention or treatment of amyotrophic lateral sclerosis (ALS) or diabetic peripheral neuropathy (DPN).

In an embodiment of the present disclosure, the diabetic peripheral neuropathy is polyneuropathy or focal neuropathy.

The pharmaceutical composition of the present disclosure uses the aforementioned AAV vector or AAV particle as the active ingredient, and therefore, the common content between the two is omitted to avoid excessive complexity in the description.

The pharmaceutically acceptable carriers included in the pharmaceutical composition of the present disclosure are those commonly used in formulation, examples of which include as lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, and mineral oil, but are not limited thereto. The pharmaceutical composition of the present disclosure may further include lubricants, wetting agents, sweeteners, flavoring agents, emulsifiers, suspending agents, preservatives, and the like. For details of suitable pharmaceutically acceptable carriers and formulations, reference may be made to Remington's Pharmaceutical Sciences (19th ed., 1995).

The pharmaceutical composition of the present disclosure can be administered orally or non-orally, for example, by intravenous administration, intrathecal administration, intramedullary administration, intracerebral administration, intraventricular administration, intracerebral parenchymal administration, intracranial administration, subcutaneous administration, intramuscular administration, intraperitoneal administration, sternal administration, local administration, intranasal administration, pulmonary administration, or rectal administration, among others. Preferably, the composition may be administered intrathecally, but with no limitations thereto.

The suitable dosage of the pharmaceutical composition of the present disclosure varies depending on factors including the formulation method, administration method, patient age, weight, gender, pathological condition, food intake, administration time, administration route, excretion rate, and response sensitivity, and a skilled physician can easily determine and prescribe the dosage effective for the desired treatment or prevention.

In a preferred embodiment of the present disclosure, the daily dosage of the pharmaceutical composition is 1×10⁹ to 1×10¹² vg/kg based on AAV viral particles. The term "pharmaceutically effective amount", as used herein, refers to an amount sufficient to prevent or treat the aforementioned diseases.

The term "prevention" as used herein refers to the prophylactic or protective treatment against a disease or disease condition. The term "treatment" as used herein refers to the reduction, inhibition, alleviation, or eradication of a disease condition.

The pharmaceutical composition of the present disclosure may be formulated into a unit dosage form or incorporated into a multi-dose container by following methods that are easily carried out by those skilled in the art using pharmaceutically acceptable carriers and/or excipients. The formulation may be in the form of a solution, suspension, or emulsion in oil or aqueous medium, or in the form of extracts, pulvis, suppositories, powders, granules, tablets, or capsules, and may additionally include dispersing agents or stabilizers.

Another aspect of the present disclosure provides a method for preventing or treating amyotrophic lateral sclerosis (ALS) or diabetic peripheral neuropathy (DPN), the method comprising a step of administering the aforementioned pharmaceutical composition to a subject in need of treatment.

In an embodiment of the present disclosure, the pharmaceutical composition includes the AAV vector, the isolated host cells containing the AAV vector, or AAV particles containing the AAV vector as an active ingredient.

The subject of the present disclosure may be a mammal, such as a human, chimpanzee, orangutan, non-human primate, dog, cat, horse, hamster, mouse, rat, gerbil, pig, sheep, cow, and the like, with the most specific example being a human, but is not limited thereto.

Since the method for preventing or treating amyotrophic lateral sclerosis (ALS) or diabetic peripheral neuropathy (DPN) includes administering the pharmaceutical composition, which is an aspect of the present disclosure, redundant content has been omitted to avoid excessive repetition in this specification.

### Advantageous Effects of Invention

The present disclosure provides an AAV vector optimized for intrathecal administration, with a hepatocyte growth factor gene introduced thereinto, AAV particles, and a pharmaceutical composition containing same for the prevention or treatment of amyotrophic lateral sclerosis (ALS) or diabetic peripheral neuropathy (DPN).

The AAV vector of the present disclosure exhibit excellent productivity of AAV particles containing the hepatocyte growth factor gene, and when administered intrathecally to mammals, the AAV particles are high in the expression efficiency of hepatocyte growth factor. Therefore, the use of the AAV vector and particles of the present disclosure for intrathecal administration induces the expression of the hepatocyte growth factor protein, thus finding advantageous applications in the prevention or treatment of related diseases.

### Brief Description of Drawings

FIG. 1 shows diagrams of the composition of the AAV vector candidates, categorized by promoter, GOI, and polyadenylation sequence, as presented in Table 1.
FIG. 2 is a graph of the hHGF expression levels compared according to the composition of the AAV vector candidates in Table 1.
FIG. 3 is a graph showing a comparison of the productivity of AAV particles produced according to the composition of the AAV vector candidates in Table 1 of the present disclosure.
FIG. 4 is a graph of the comparison of hHGF expression levels after intrathecal administration to mice of AAV particles produced according to the composition of the AAV vector candidates in Table 2.

### Best Mode for Carrying out the Invention

Hereinafter, embodiments of the disclosure will be described in detail with reference to the accompanying drawings. In the following description of the disclosure, a detailed description of known functions or ~ A better understanding of the present disclosure may be obtained through the following examples, which are set forth to illustrate, but are not to be construed to limit, the present disclosure. It would be obvious to those of ordinary skill in the art that the claims of the present disclosure are not limited to such examples.

### EXAMPLES

Throughout the description, the term "%" used to express the concentration of a specific material, unless otherwise particularly stated, refers to (wt/wt) % for solid/solid, (wt/vol) % for solid/liquid, and (vol/vol) % for liquid/liquid.

### EXAMPLE 1: Construction of Candidate Plasmids and Comparison of Expression Efficiency (in vitro)

Six types of plasmids, each containing different promoters and poly A tails, were constructed through cloning as follows:
(1) A backbone sequence (SEQ ID NO: 20) including ITR(L) and ITR(R) was obtained through synthesis.
(2) The CMV promoter (SEQ ID NO: 11) and EF-1alpha S promoter (SEQ ID NO: 12) were each synthesized to include MluI and ClaI restriction enzyme sequences and inserted into the backbone.
(3) The HGF-X7-d4 sequence of SEQ ID NO: 8 was inserted using ClaI and SalI restriction enzyme sequences.
(4) The poly A tail candidate sequences-hGH poly A tail (SEQ ID NO: 14), bGH poly A tail (SEQ ID NO: 16), and SV40 poly A (SEQ ID NO: 18)-were each synthesized to include SalI and RsrII restriction enzyme sequences and inserted into the backbone.

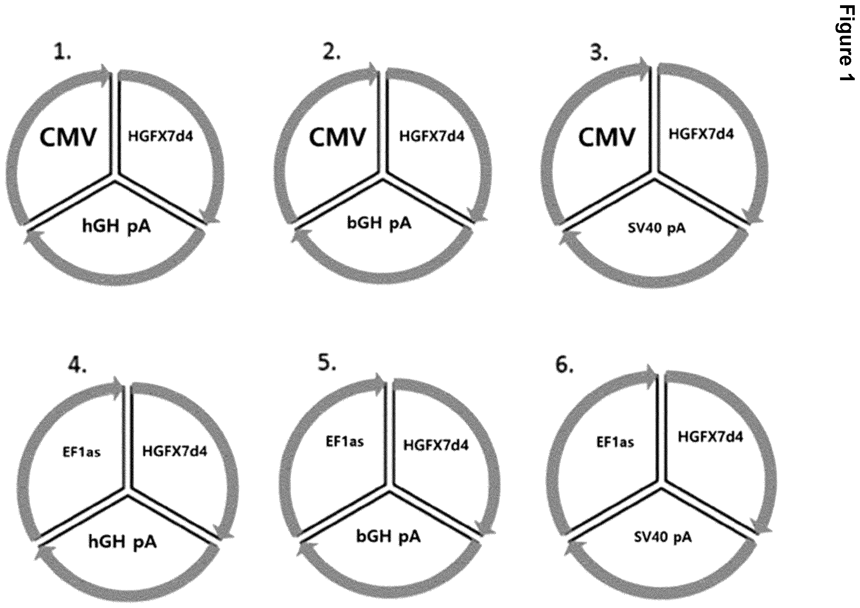

The composition of the six plasmids is shown in Table 1 and FIG. 1. As a positive control, VM202 (pCK-HGF-X7) was used.

**TABLE 1**

| No . | Promoter | Expressed gene | Poly A tail |
|---|---|---|---|
| 1 | CMV (1160 bp) | HGFX7d4 (2879 bp) | hGH pA (542 bp) |
| 2 | CMV (1160 bp) | HGFX7d4 (2879 bp) | bGH pA (317 bp) |
| 3 | CMV (1160 bp) | HGFX7d4 (2879 bp) | SV40 pA (185 bp) |
| 4 | EF-1alpha S (784 bp) | HGFX7d4 (2879 bp) | hGH pA (542 bp) |
| 5 | EF-1alpha S (784 bp) | HGFX7d4 (2879 bp) | bGH pA (317 bp) |
| 6 | EF-1alpha S (784 bp) | HGFX7d4 (2879 bp) | SV40 pA (185 bp) |

FIG. 1 shows schematic diagrams of the composition of the AAV vector candidates, categorized by promoter, GOI, and polyadenylation sequence, as presented in Table 1.

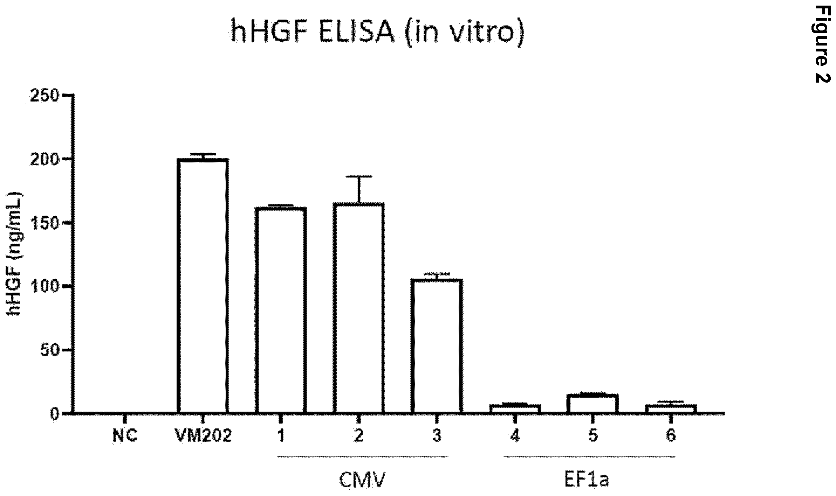

HEK293 cells were cultured in DMEM + 10% FBS medium. One day before transfection, the cells were seeded at a density of 1×10⁵ cells/well in 6-well plates. The following day, NC (negative control), VM202 (positive control), and Plasmids 1 through 6 (0.5 µg each) were added, and transfection was performed using PEI. After 72 hours, the supernatant was collected, and human HGF ELISA (R&D, DHG00) was conducted. The results are shown in FIG. 2. FIG. 2 is a graph of the hHGF expression levels compared according to the composition of the AAV vector candidates in Table 1.

As shown in FIG. 2, hHGF was well expressed in the positive control VM202. Plasmids 1 through 3, each containing a CMV promoter, showed relatively high levels of hHGF expression, with a lower expression level for plasmid 3 containing SV40 polyA than for plasmid 1 or 2. Plasmids 4 through 6, each containing the EF-1alpha S promoter, showed significantly lower hHGF expression levels.

### EXAMPLE 2: Comparison of AAV Productivity Among Candidate Plasmids (in vitro)

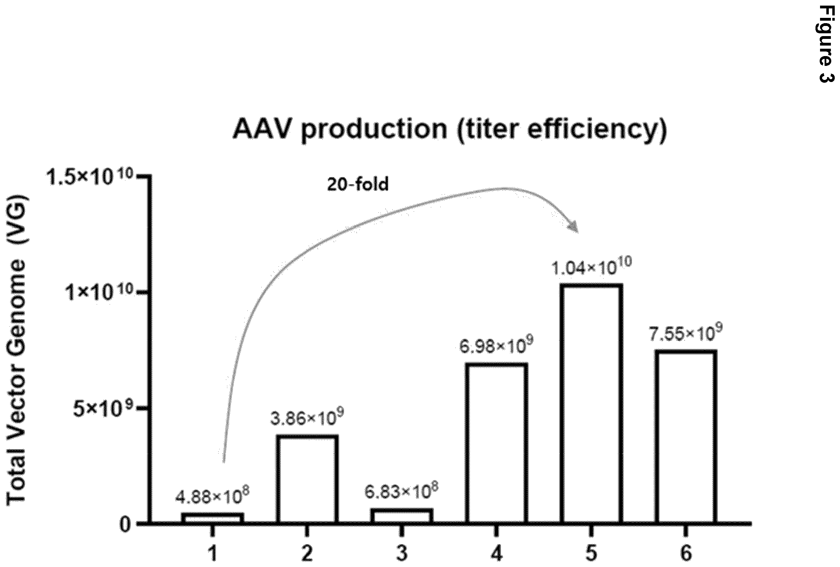

AAV vectors were prepared using the six types of plasmids from Example 1. First, HEK293 cells were seeded into T225 flasks and co-transfected with three plasmids (pAAV-RC, pAAV-helper, and pAAV-HGF) with the aid of PEI. After 72 hours, the cells were collected, and AAV purification was performed, followed by titration of the AAV. The results are shown in FIG. 3. FIG. 3 is a graph of the productivity of AAV particles produced according to the composition of the AAV vector candidates in Table 1 of the present disclosure.

As shown in FIG. 3, plasmids 1 and 3, which included CMV promoters, showed the lowest levels of AAV productivity. Among the plasmids including CMV promoters, plasmid 2 with a bGH polyA tail demonstrated the best AAV productivity.

In contrast, plasmids 4 through 6, which included EF-1alpha promoters, exhibited relatively higher AAV productivity compared to the plasmids containing CMV promoters. Notably, plasmid 5 showed approximately 20 times higher AAV production compared to plasmid 1.

### EXAMPLE 3: Comparison of hHGF Expression Levels of Candidate Plasmids (in vivo)

AAV1 or AAV9 were provided using plasmids 1, 4, and 5, which were constructed in the previous examples, and plasmid 11, which is a variant of plasmid 5 in which the expressed gene was replaced with a codon-optimized HGFX7d4. These particles were administered to mice to compare the expression levels of hHGF protein (flHGF) under *in vivo* conditions.

**TABLE 2**

| Group | AAV | Promoter | Expressed gene | Poly A |
|---|---|---|---|---|
| Sham | - | - | - | - |
| AAV1_#1 | AAV1 | CMV (1160 bp) | HGFX7d4 (2879 bp) | hGH pA (542 bp) |
| AAV9_#1 | AAV9 | CMV (1160 bp) | HGFX7d4 (2879 bp) | hGH pA (542 bp) |
| AAV1_#4 | AAV1 | EF-1alpha S (784 bp) | HGFX7d4 (2879 bp) | hGH pA (542 bp) |
| AAV1_#5 | AAV1 | EF-1alpha S (784 bp) | HGFX7d4 (2879 bp) | bGH pA (317 bp) |
| AAV1_#11_codon opti | AAV1 | EF-1alpha S (784 bp) | HGFX7d4 codon opti (2879 bp) | bGH pA (317 bp) |
| AAV9_#11_codon opti | AAV9 | EF-1alpha S (784 bp) | HGFX7d4 codon opti (2879 bp) | bGH pA (317 bp) |

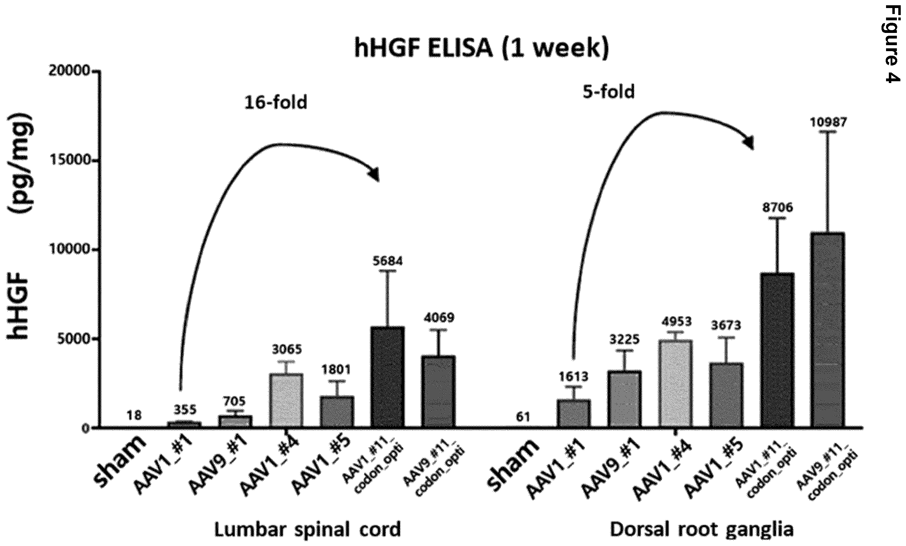

The AAV (1×10¹⁰ vg/head) under the conditions in Table 2 was administered intrathecally to C57BL/6 mice. Seven days later, the lumbar spinal cord and dorsal root ganglia (DRG) regions were collected. The collected tissue samples were processed with RIPA buffer to extract proteins, and the total protein amount was quantified using the BCA Protein Assay kit (Thermo, Cat# 23225). Subsequently, hHGF protein expression levels were compared using a Human HGF Quantikine ELISA kit (R&D, DHG00). The spinal cord and DRG lysates were each added in an amount 50 µL per well and mixed with antibodies. A standard curve was drawn using the Human HGF standard provided in the ELISA kit, and the hHGF amount in the final samples was derived based on this curve. The results are shown in FIG. 4. FIG. 4 is a graph of the comparison of hHGF expression levels after intrathecal administration to mice of AAV particles produced according to the composition of the AAV vector candidates in Table 2.

As shown in FIG. 4, contrary to expectations, AAVs with EF-1alpha promoters overall expressed higher levels of hHGF compared to AAVs with CMV promoters. This is notable because the results are opposite to those observed *in vitro.* Particularly, the candidate with the codon-optimized HGF-X7-d4 under the EF-1alpha promoter exhibited significantly higher levels of hHGF expression. Quantitative comparison revealed that AAV_#11_codon_\opti expressed approximately 16 times more hHGF in the spinal cord and about 5 times more in the DRG compared to AAV_#1.

Based on these test results, plasmid 11, which showed high AAV productivity and *in vivo* hHGF expression, was selected as the final candidate.

The foregoing details are provided for the convenience of understanding of the disclosure, and the disclosure is not limited thereto.

## Claims

1. An adeno-associated virus (AAV) vector, carrying a nucleic acid molecule comprising:
(a) a Cytomegalovirus (CMV) promoter sequence or an elongation factor 1-alpha (EF-1alpha) promoter sequence; and
(b) a polynucleotide sequence encoding hepatocyte growth factor (HGF) operatively linked to the promoter sequence.

2. The AAV vector of claim 1, wherein the hepatocyte growth factor is a hepatocyte growth factor (HGF) isoform.

3. The AAV vector of claim 2, wherein the hepatocyte growth factor isoform is a full-length hepatocyte growth factor (flHGF), a deleted variant hepatocyte growth factor (dHGF), or a combination thereof.

4. The AAV vector of claim 3, wherein the full-length hepatocyte growth factor comprises the amino acid sequence of SEQ ID NO: 1, and the deleted variant hepatocyte growth factor comprises the amino acid sequence of SEQ ID NO: 2.

5. The AAV vector of claim 1, wherein the polynucleotide sequence encoding hepatocyte growth factor has an identity of at least 95% with a nucleotide sequence selected from the group consisting of the nucleotide sequences of SEQ ID NOS: 4 to 9 and 19.

6. The AAV vector of claim 1, wherein the polynucleotide sequence encoding hepatocyte growth factor comprises the nucleotide sequence of SEQ ID NO: 8 or 9.

7. The AAV vector of claim 1, wherein the AAV vector has a serotype selected from the group consisting of AAV1, AAV2, AAV4, AAV5, AAV6, AAV8, AAV9, AAV10, AAV11, AAV12, AAV13, AAV-DJ, AAV-DJ/8, AAV-PHP.Eb, AAV-PHP.S, and AAV rh10.

8. The AAV vector of claim 1, wherein the AAV vector has a serotype of AAV1 or AAV9.

9. The AAV vector of claim 1, wherein the CMV promoter sequence comprises the nucleotide sequence of SEQ ID NO: 10 or 11.

10. The AAV vector of claim 1, wherein the EF-1alpha promoter sequence comprises the nucleotide sequence of SEQ ID NO: 12.

11. The AAV vector of claim 1, wherein the nucleic acid molecule further comprises a polyadenylation sequence.

12. The AAV vector of claim 11, wherein the polyadenylation sequence comprises an SV40 polyadenylation sequence, a human growth hormone (hGH) polyadenylation sequence, a bovine growth hormone (bGH) polyadenylation sequence, or a combination thereof.

13. The AAV vector of claim 1, wherein the nucleic acid molecule comprises two AAV inverted terminal repeat sequences (ITR sequences) flanking both sides of the nucleic acid sequence.

14. An isolated host cell, comprising the AAV vector of any one of claims 1 to 13.

15. An AAV particle, comprising the AAV vector of any one of claims 1 to 13.

16. The AAV particle of claim 15, wherein the AAV particle is for intrathecal administration.

17. A pharmaceutical composition for the prevention or treatment of amyotrophic lateral sclerosis (ALS) or diabetic peripheral neuropathy (DPN), the composition comprising the AAV particle of claim 15.

18. The pharmaceutical composition of claim 17, wherein the diabetic peripheral neuropathy is polyneuropathy or focal neuropathy.
